(19) Europäisches Patentamt
European Patent Office
Office européen des brevets

(11) **EP 3 629 261 A1**

(12) **EUROPEAN PATENT APPLICATION**

(43) Date of publication:
**01.04.2020 Bulletin 2020/14**

(51) Int Cl.:
*G06Q 10/06* (2012.01)      *G01N 33/00* (2006.01)

(21) Application number: **18196994.0**

(22) Date of filing: **26.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Valeo Systemes Thermiques-THS 78322 Le Mesnil Saint Denis Cedex (FR)**

(72) Inventors:
• **DE PELSEMAEKER, Georges**
  **78322 LE MESNIL SAINT DENIS CEDEX (FR)**
• **CLEMARON, Laetitia**
  **78322 LE MESNIL SAINT-DENIS CEDEX (FR)**
• **DEVEYCX, Marion**
  **78322 LE MESNIL SAINT-DENIS (FR)**

(74) Representative: **Metz, Gaëlle**
  **Valeo Systèmes Thermiques**
  **8, rue Louis Lormand**
  **CS 80517 La Verrière**
  **78322 Le Mesnil Saint Denis Cedex (FR)**

(54) **A COMPUTER-IMPLEMENTED METHOD AND A SYSTEM FOR CALIBRATION OF VEHICLE POLLUTION SENSORS IN A VEHICLE AIR QUALITY SYSTEM**

(57)     A computer-implemented method for calibration of vehicle pollution sensors in a vehicle air quality system, comprising steps of: obtaining navigation data, indicative of vehicle position; obtaining context pollution data from a data provider, indicative of pollution levels within a defined area in which a vehicle is located; obtaining sensor measurement pollution data from a vehicle pollution sensor; comparing sensor measurement pollution data and context pollution data for current vehicle position to determine discrepancies in comparable values; adjusting the sensor pollution data in view of the discrepancies; outputting the adjusted sensor measurement pollution data.

Fig. 2

## Description

### FIELD OF THE INVENTION

The invention relates to a computer-implemented method for calibration of vehicle pollution sensors in a vehicle air quality system. It also relates to a system for calibration of vehicle pollution sensors in a vehicle air quality system.

### BACKGROUND OF THE INVENTION

Air pollution is a serious concern for people because of its harmful effects to health - both in physical and psychological sense. Passengers travelling in vehicles are especially at risk, as the vehicles are part of traffic existing in urbanized areas, which tend to have higher concentration of air pollution and higher levels of pollutant gases and particles.

One way of remedying this disadvantageous situation is development of cabin air quality systems in order to prevent or limit exposure of vehicle passengers to polluted air.

Pollution levels are monitored by government agencies and private entities. The measurements are often provided in form of so called pollution maps. For example, a layer of visually represented information is added to a classic map, so that pollution concentration within a specific area or region can be determined and evaluated. Such maps usually have low time-resolution, which means that the information is updated relatively rarely, for example once an hour or once a day. Pollution map data is obtained for example by measuring pollution with a number of measuring stations or pollution measurement devices of a fixed position.

Further, vehicles can be equipped with pollution sensors and be adapted to provide pollution level values based on real-time and/or aggregated measurements, be it inside the vehicle cabin or outside of the vehicle, in its immediate vicinity.

The sensors which are installed in vehicles may be subject to degradation of their operation with time, i.e. to ageing and wear. Similarly, some sensors may become damaged or exhibit manufacturing defects only after some period of normal operation. The effects of such occurrence may not become visible soon enough. For example, a pollution sensor may output measurement values which do not reflect the actual pollution level, but which at the same time do not exhibit deviation from standard measurement values which would prompt immediate attention of the system or the user. This may be especially dangerous for the health of passengers if the sensor outputs measurement values of the pollution level lower than the actual pollution level. Effectively, the vehicle air quality system may adapt its operation as if the pollution was smaller than it actually is, and unnecessarily expose the passengers.

It would be thus advantageous to provide a method and a system which would allow to detect the above-mentioned situation and enable counter-action either by the vehicle systems or by the passengers of the vehicle themselves.

### SUMMARY OF THE INVENTION

The object of the invention is, among others, a computer-implemented method for calibration of vehicle pollution sensors in a vehicle air quality system, comprising steps of: obtaining navigation data, indicative of vehicle position; obtaining context pollution data from a data provider, indicative of pollution levels within a defined area in which a vehicle is located; obtaining sensor measurement pollution data from a vehicle pollution sensor; comparing sensor measurement pollution data and context pollution data for current vehicle position to determine discrepancies in comparable values; adjusting the sensor pollution data in view of the discrepancies; outputting the adjusted sensor measurement pollution data.

Preferably, the context pollution data is obtained from a pollution map.

Preferably, the sensor pollution data comprises pollution data indicative of pollution levels outside the vehicle, in its immediate vicinity.

Preferably, upon detecting discrepancy level associated with sensor failure, operation of the vehicle pollution sensor is disabled.

Preferably, upon detecting discrepancy level associated with sensor failure, the adjusted sensor pollution data is equalized with the context pollution data.

Preferably, sensor measurement pollution data and context pollution data values are AQI values.

Preferably, sensor measurement pollution data and context pollution data values are pollutant concentration values measured in ppm, ppb or $\mu g/m3$.

Preferably, at least the steps of obtaining context pollution data from a data provider, comparing sensor measurement pollution data and context pollution data for current vehicle position to determine discrepancies in comparable values; adjusting the sensor pollution data in view of the discrepancies; and outputting the adjusted sensor measurement pollution data are performed by means of cloud computing.

The object of the invention is also a system for calibration of vehicle pollution sensors in a vehicle air quality system, comprising: communication means configured to provide context pollution data, indicative of pollution levels within a defined area in which a vehicle is located, and navigation data, representative of geographical position of the vehicle; pollution sensing means adapted to be mounted on a vehicle, comprising a pollution sensor, configured to provide sensor measurement pollution data; control means configured to determine discrepancy level between comparable pollution measurement values of the context pollution data and sensor measurement pollution data and an action associated with the determined discrepancy level; output means configured to execute the determined action.

Preferably, the pollution sensing means comprise a sensor configured to measure pollution levels outside the vehicle, in its immediate vicinity.

Preferably, the communication means are adapted to provide internet connection for the system, in particular to cloud computing services.

Preferably, the communication means are adapted to communicate with other systems according to the invention and exchange data.

The object of the invention is also a computer readable storage medium storing a program of instructions executable by a machine to perform a method as described above.

## BRIEF DESCRIPTION OF DRAWINGS

Examples of the invention will be apparent from and described in detail with reference to the accompanying drawings, in which:

> Fig. 1 shows a flowchart presenting the method according to the invention;

> Fig. 2 shows a first exemplary plot presenting context and sensor data;

> Fig. 3 shows a schematic representation of a system according to invention.

## DETAILED DESCRIPTION OF EMBODIMENTS

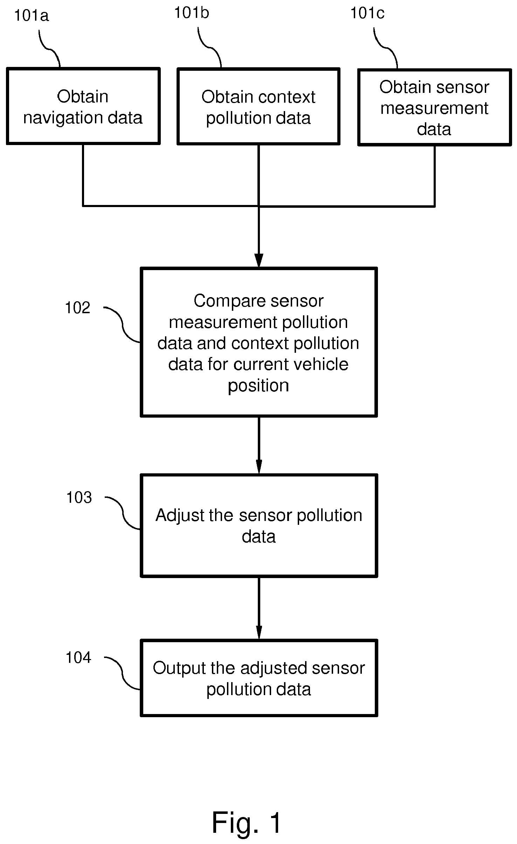

Fig. 1 shows a flowchart presenting the method according to the invention.

Firstly, the method comprises 101a obtaining navigation data, indicative of vehicle position. The position of the vehicle is determined, and preferably kept updated with a predefined frequency. This is especially the case if the vehicle is moving.

In the next step, the method comprises obtaining 101b context pollution data from a data provider, indicative of pollution levels within a defined area in which a vehicle is located. The perimeter of the area is defined based on existing conditions, and the requirements of the system. The context pollution data can be a pollution map data, which is indicative of air pollution levels within the specified area. Such pollution map data can comprise information on particulate matter levels, carbon monoxide, sulfur oxides, nitrogen oxides, ozone, spread over a defined geographical area. It can also relate to AQI value calculated for the specified area.

In the next step, the method comprises obtaining 101c sensor measurement pollution data from a vehicle pollution sensor. This can be a single sensor or a plurality of sensors. The measurements can relate to pollution level outside the vehicle, in its immediate vicinity.

It is to be noted that the sequence of steps 101a, 101b and 101c is interchangeable. However, preferably, the navigation data is obtained before the context pollution data in order to limit the processing of unnecessary information.

In the next step, the method comprises comparing 102 sensor measurement pollution data and context pollution data for current vehicle position to determine discrepancies in comparable values, relating to the same or similar pollutants or AQI values. Further, upon detecting discrepancy level associated with sensor failure, certain actions can be executed with respect to the sensor or the system.

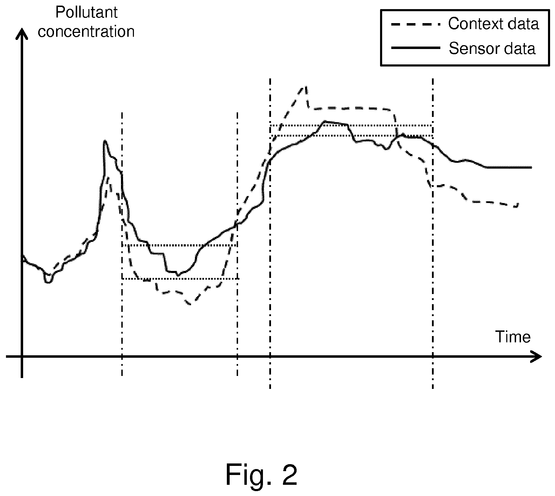

Next, the method comprises adjusting 103 the sensor pollution data in view of the discrepancies. The adjustment is explained further in relation to Fig 2 below. The object of the adjustment is to modify the measurement values from the sensor so that they are in line with the values obtained from the context pollution data. In particular, any drift, systematic or random errors of sensors can be corrected by using the information on discrepancies with respect to context pollution data. Alternatively, upon detecting discrepancy level associated with sensor failure, operation of the vehicle pollution sensor can disabled, and the adjusted sensor pollution data can be produced based only on the context pollution data. For example, the adjusted sensor pollution data can be equalized with the context pollution data.

In the next step, the method comprises outputting 104 the adjusted sensor measurement pollution data. The outputting 104 can be carried out by direct feeding of the adjusted sensor measurement pollution data into the vehicle air quality system instead of the sensor measurement pollution data.

It is to be noted that any measurements taken when the navigation data is incomplete (insufficient) or missing, for example in situations when the car is in a tunnel, garage or shopping mall parking, will not be treated like those with sufficient navigation data. These measurements can be either ignored or estimated based on a derived model. The system can return to normal operation once the up-to-date navigation data is available again.

The above-described method can be carried out by means of cloud computing, in cooperation with a vehicle air quality system. For example, the vehicle air quality system can be adapted to obtain the sensor measurement pollution data and navigation data and send it to the cloud, and subsequently receive the outputted adjusted sensor measurement pollution data from said cloud. In other words, at least the steps 101b, 102, 103 and 104 can be performed within the cloud.

Fig. 2 shows a first exemplary plot presenting context and sensor data. A context pollution data is plotted together with sensor measurement pollution data, i.e. the comparable values thereof. In this example, the pollution concentration values are measured for a period of time. The pollutants can be PM1, PM2.5, PM10, NOx, CO, CO2, SO2, Ozone, VOC (*Volatile Organic Compounds*), allergens and others. It is also envisaged that the sensor unit is able to calculate AQI (*Air Quality Index*) by itself thanks to an on-board micro-controller. In such case, an AQI value is outputted as a measurement. Such AQI value may be adjusted analogously to the direct sensor measurement pollution data values. In any case, the values can be averaged for selected duration, as presented in Fig. 2. Then, the sensor measurement pollution data can be adjusted with a function of the context pollution data by identification of parameters α and β:

$$Average_{context} = \alpha.Average_{sensor} + \beta$$

After obtaining the parameters α and β, the values of sensor measurement pollution data can be adjusted:

$$Sensor_{adjusted} = \alpha.Sensor_{measured} + \beta$$

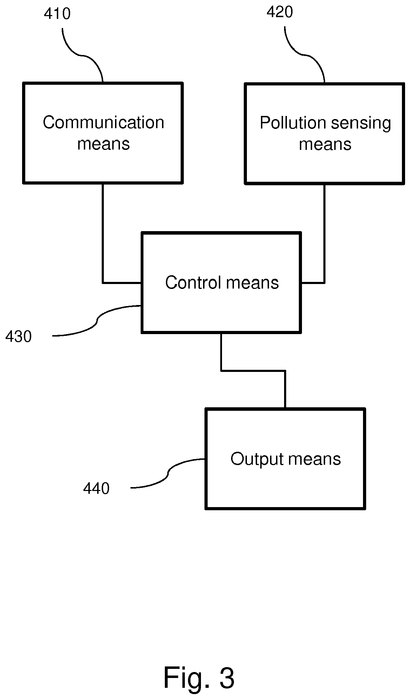

Fig. 3 presents a schematic representation of a system according to invention. The system comprises communication means 410, pollution sensing means 420, control means 430 and output means 440.

The communication means 410 are adapted to receive and send wireless signals, such as navigation signals, for example GPS signals or the like. In particular, the communication means 410 are adapted to provide context pollution data, indicative of pollution levels within a defined area in which a vehicle is located, and navigation data, representative of geographical position of the vehicle. The communication means 410 can be adapted to provide internet connection to the system, in particular to cloud computing services. All received data is supplied to and from control means 430. The communication means 410 may be also adapted to communicate with other vehicles comprising system according to the invention, for example via internet or by other means, so that data can be exchanged.

The pollution sensing means 420 comprise pollution sensors which measure air pollution levels outside the vehicle, in its immediate vicinity.

The control means 430 are adapted to receive data from pollution sensing means 420 as well as from the communication means 410. In particular, the control means 430 are adapted to obtain: navigation data, indicative of vehicle position; context pollution data from a data provider, indicative of pollution levels within a defined area in which a vehicle is located; sensor measurement pollution data from a vehicle pollution sensor. The control means 430 are further configured to compare sensor measurement pollution data and context pollution data for current vehicle position to determine discrepancies in comparable values and to adjust the sensor pollution data in view of the discrepancies. The control means 430 can comprise memory for storing data regarding discrepancy levels and actions associated with said discrepancy levels.

The system further comprises output means 440. These are adapted to output the adjusted sensor measurement pollution data.

It is envisaged that the communication means 410 are also adapted to obtain supplemental data from other vehicles with the system according to the invention. This might be the case especially for vehicles with identical or similar configuration, identical or similar age, and/or operating in the same or similar area, subject to the same or similar pollution levels. Such pool can be used to further improve adjustment between context pollution data and sensor measurement data. Thanks to data sent to a cloud it is possible to build a data base for every embedded sensors equipped on certain vehicle model in a specific area. With this data base, it is possible to derive information from sensors behavior regarding ageing, and then anticipate the required correction to obtain a value closer to the value obtained from the pollution map.

The invention as presented above allows to counter-act measurement errors produced by on-board pollution sensors. It enables utilization of big data related to pollution levels to recalibrate sensors by means of data analysis and correction algorithms. Dismounting of the sensors for maintenance or replacement during car servicing can thus be avoided or reduced.

It is understood that embodiments of the present invention can be carried out in form of hardware, software or a combination of both. Any such software can be stored in the form of a volatile or non-volatile storage, for example a storage like a ROM, whether erasable or rewritable or not, or in the form of memory such as, for example RAM, memory chips, device or integrated circuits or an optically or magnetically readable medium such as, for example, a CD, DVD, magnetic disk or magnetic tape. It is understood that the storage devices and storage media are embodiments of machine-readable storage that are suitable for storing program or programs that, when executed, implement embodiments of the present invention. Accordingly, embodiments provide a program comprising code for implementing a system or method as described and a machine readable storage storing such a program. Further, embodiments of the present invention may be conveyed electronically via any medium such as a communication signal carried over a wired or wireless connection and embodiments suitably encompass the same.

Other variations to the disclosed embodiments can be understood and effected by those skilled in the art in practicing the claimed invention, from a study of drawings, the disclosure, and the appended claims. The mere fact that certain measures are recited in mutually different dependent claims does not indicate that a combination of these measures cannot be used to the advantage.

**Claims**

1. A computer-implemented method for calibration of vehicle pollution sensors in a vehicle air quality system, comprising steps of:

   - obtaining 101a navigation data, indicative of vehicle position;
   - obtaining 101b context pollution data from a data provider, indicative of pollution levels within a defined area in which a vehicle is located;
   - obtaining 101c sensor measurement pollution data from a vehicle pollution sensor;
   - comparing 102 sensor measurement pollution data and context pollution data for current vehicle position to determine discrepancies in comparable values;
   - adjusting 103 the sensor pollution data in view of the discrepancies;
   - outputting 104 the adjusted sensor measurement pollution data.

2. A method according to claim 1, wherein the context pollution data is obtained from a pollution map.

3. A method according to any preceding claim, wherein the sensor pollution data comprises pollution data indicative of pollution levels outside the vehicle, in its immediate vicinity.

4. A method according to any preceding claim, wherein upon detecting discrepancy level associated with sensor failure, operation of the vehicle pollution sensor is disabled.

5. A method according to any preceding claim, wherein upon detecting discrepancy level associated with sensor failure, the adjusted sensor pollution data is equalized with the context pollution data.

6. A method according to any preceding claim, wherein sensor measurement pollution data and context pollution data values are AQI values.

7. A method according to any preceding claim, wherein sensor measurement pollution data and context pollution data values are pollutant concentration values measured in ppm, ppb or $\mu$g/m3.

8. A method according to any preceding claims, wherein at least the steps 101b, 102, 103 and 104 are performed by means of cloud computing.

9. A system for calibration of vehicle pollution sensors in a vehicle air quality system, comprising:

   - communication means 410 configured to provide context pollution data, indicative of pollution levels within a defined area in which a vehicle is located, and navigation data, representative of geographical position of the vehicle;
   - pollution sensing means 420 adapted to be mounted on a vehicle, comprising a pollution sensor, configured to provide sensor measurement pollution data;
   - control means 430 configured to determine discrepancy level between comparable pollution measurement values of the context pollution data and sensor measurement pollution data and an action associated with the determined discrepancy level;
   - output means 440 configured to execute the determined action.

10. A system according to claim 9, wherein the pollution sensing means 420 comprise a sensor configured to measure pollution levels outside the vehicle, in its immediate vicinity.

11. A system according to any of claims 9 to 10, wherein the communication means 410 are adapted to provide internet connection for the system, in particular to cloud computing services.

12. A system according to any of claims 9 to 11, wherein the communication means 410 are adapted to communicate with other systems according to the invention and exchange data.

13. A computer readable storage medium storing a program of instructions executable by a machine to perform a method according to any of claims 1-8.

| | | |
|---|---|---|
| 101a | 101b | 101c |
| Obtain navigation data | Obtain context pollution data | Obtain sensor measurement data |

102 — Compare sensor measurement pollution data and context pollution data for current vehicle position

103 — Adjust the sensor pollution data

104 — Output the adjusted sensor pollution data

Fig. 1

Fig. 2

410

420

Communication means

Pollution sensing means

Control means

430

Output means

440

Fig. 3

Europäisches
Patentamt

European
Patent Office

Office européen
des brevets

# EUROPEAN SEARCH REPORT

Application Number

EP 18 19 6994

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/202824 A1 (BORREL HERVE [US]) 19 July 2018 (2018-07-19) * paragraph [0017] - paragraph [0026] * * paragraph [0037] - paragraph [0047] * * paragraph [0061]; figures 3,5 * ----- | 1-13 | INV. G06Q10/06 G01N33/00 |
| X | WILL HEDGECOCK ET AL: "Dissemination and presentation of high resolution air pollution data from mobile sensor nodes", 20100415; 20100415 - 20100417, 15 April 2010 (2010-04-15), pages 1-6, XP058103269, DOI: 10.1145/1900008.1900019 ISBN: 978-1-4503-0064-3 * the whole document * ----- | 1-13 | |

TECHNICAL FIELDS
SEARCHED     (IPC)

G06Q
G01N

The present search report has been drawn up for all claims

| Place of search | Date of completion of the search | Examiner |
|---|---|---|
| The Hague | 29 October 2018 | Moynihan, Maurice |

EPO FORM 1503 03.82 (P04C01)

**ANNEX TO THE EUROPEAN SEARCH REPORT
ON EUROPEAN PATENT APPLICATION NO.**

EP 18 19 6994

This annex lists the patent family members relating to the patent documents cited in  the above-mentioned European search report.
The members are as contained in the European Patent Office EDP file on
The European Patent Office is in no way liable for these particulars which are merely given for the purpose of information.

29-10-2018

| Patent document cited in search report | Publication date | Patent family member(s) | Publication date |
|---|---|---|---|
| US 2018202824 A1 | 19-07-2018 | NONE | |

EPO FORM P0459

For more details about this annex : see Official Journal of the European Patent Office, No. 12/82